# FASCICULE DE BREVET EUROPEEN

(11) **EP 0 365 412 B1**
(45) Date de publication et mention de la délivrance du brevet: **17.05.1995**
(21) Numéro de dépôt: 89402844.8
(22) Date de dépôt: 13.10.1989
(51) Int. Cl.: C07D 257/02, C07F 5/00, A61K 49/04

(54) **Nouveaux ligands cycliques azotés, complexes métalliques formés par ces ligands, compositions de diagnostic contenant ces complexes et procédé de préparation des ligands**
Zyklische, Stickstoff enthaltende Liganden, ihre Metallkomplexe, diese enthaltende Diagnostik-Mittel und Verfahren zur Herstellung der Liganden
Cyclic nitrogen-containing ligands, their metal complexes, diagnostic compositions containing these complexes and method of preparing the ligands

(30) Priorité: 14.10.1988 FR 8813585
(43) Date de publication de la demande: 25.04.1990
(73) Titulaire: GUERBET S.A., F-93420 Villepinte (FR)
(72) Inventeur: Schaefer, Michel, F-91380 Chilly-Mazarin (FR); Paris, Dominique, F-93600 Aulnay-sous-Bois (FR); Meyer, Dominique, F-75013 Paris (FR); Doucet, Didier, F-93190 Livry-Gargan (FR)
(74) Mandataire: Le Guen, Gérard

(56) Documents cités:
- EP-A- 232 751
- EP-A- 287 465
- EP-A- 0 255 471
- EP-A- 0 292 689
- WO-A-86/02352
- WO-A-87/06229
- M. Hediger, Helvetica Chimica Acta, 66(3)(1983), 861-870
- J.S.Bradshaw, J.Org.Chem, 1988, 53, 3190-3195
- D. Parker, Pure & Appl. CHem., 61(9), 1637-1641 (1989)

## Description

La présente invention concerne de nouveaux ligands cycliques azotés et des complexes métalliques formés par ces ligands, les applications de ces complexes en imagerie par résonance magnétique, en radiologie par rayons X et comme agents de déplacement chimique in vivo.

L' invention concerne également un procédé de préparation de ces ligands.

**EP-A-0255 471** donne une formule générale de macrocycles azotés et les complexes métalliques formés avec ces composés et des ions paramagnétiques qui sont utiles dans le diagnostic par imagerie par résonance magnétique nucléaire.

**EP-A-0 287 465**, document opposable seulement au titre de l'Article 54(3) CBE, décrit des macrocycles azotés, mais sans groupes amides.

WO 86/02352 décrit des chélates de gadolinium avec des macrocycles comme l'acide 1,4,7,10-tétraazacyclododécane-N,N',N'',N'''-tetraacétique.

L'invention a ainsi pour objet des ligands de formule 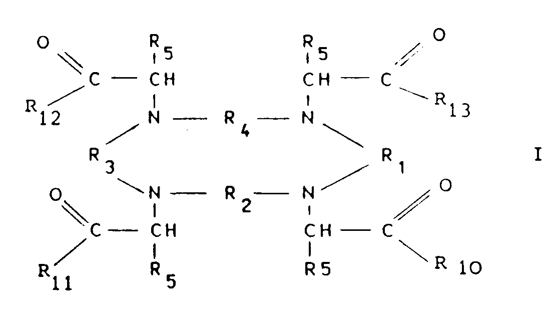
dans laquelle
R₁ représente un radical de formule 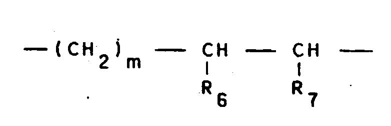
R₆ étant choisi parmi un groupe alkyle en C₁-C₁₈, un groupe hydroxyalkyle en C₁-C₆ et un groupe polyhydroxyalkyle en C₁-C₆
m = 0 ou 1,
R₇ étant choisi parmi un atome d'hydrogène, un groupe alkyle en C₁-C₁₄, un groupe hydroxyalkyle en C₁-C₆ et un groupe polyhydroxyalkyle en C₁-C₆,
R₂, R₃, R₄ identiques ou différents représentent un radical de formule 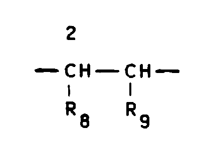
R₈, R₉ identiques ou différents étant choisis parmi un atome d'hydrogène, un groupe alkyle en C₁-C₁₄, une groupe hydroxalkyle en C₁-C₆ et un groupe polyhydroxyalkyle en C₁-C₆,
R₅ est choisi parmi un atome d'hydrogène, un groupe alkyle en C₁-C₁₄, un groupe hydroxyalkyle en C₁-C₆ et un groupe polyhydroxyalkyle en C₁-C₆,
R₁₀, R₁₁, R₁₂, R₁₃ sont choisis indépendamment l'un de l'autre parmi les groupes hydroxy et les groupes de formule 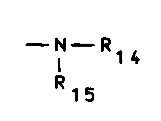
R₁₄ et R₁₅ identiques ou différents étant choisis parmi un atome d'hydrogène, un groupe alkyle en C₁-C₁₄, une groupe hydroxalkyle en C₁-C₆ et un groupe polyhydroxyalkyle en C₁-C₆,
au moins l'un des groupes R₁₀, R₁₁, R₁₂ et R₁₃ étant un groupe de formule 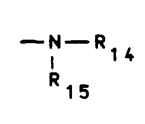
ainsi que leurs sels.

Les ligands de formule I peuvent être préparés par réaction des composés de formule 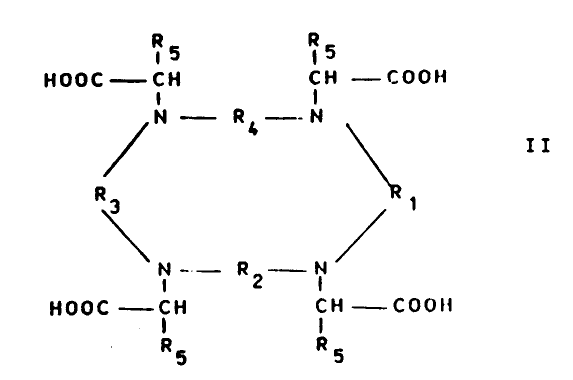
dans laquelle R₁, R₂, R₃, R₄, R₅ ont la signification donnée précédemment, avec une amine de formule 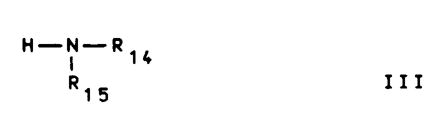
R₁₄ et R₁₅ ayant la signification donnée précédemment.

La réaction peut être effectuée notamment en milieu aqueux en présence d'un réactif de couplage tel que ceux utilisé pour la synthèse de peptides, et éventuellement en présence de catalyseurs tels que des dérivés de pyridine.

Les composés de formule II peuvent être préparés par réaction d'un composé de formule 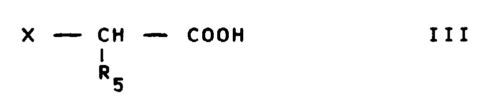
dans laquelle R₅ à la signification donnée ci-dessus et X représente un groupe labile tel qu'un atome de chlore, de brome ou d'iode ou un groupe tosyloxy ou mésyloxy, avec une amine cyclique de formule 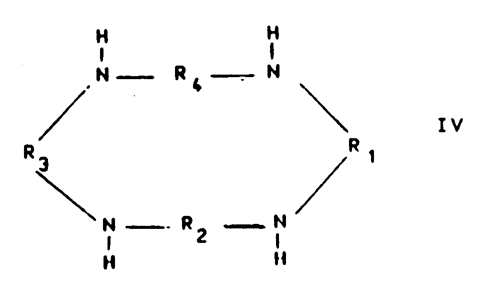
dans laquelle R₂, R₃, R₄, ont la signification donnée précédemment.

Les ligands de formule II peuvent être également préparés selon une réaction de Strecker, par réaction sur une amine cyclique de formule IV d'un aldéhyde de formule.

R₅―CHO V

dans laquelle R₅ a la signification donnée précédemment, en présence d'acide cyanhydrique ou plus généralement d'ions cyanure (KCN, NaCN).

Les composés de formule IV peuvent être préparés
a) par réaction d'une polyamine de formule 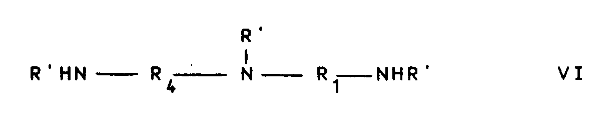 dans laquelle R₁ et R₄ ont la signification donnée précédemment et R′ représente un groupe tosyle, mésyle ou benzène sulfonyle
   avec un composé de formule 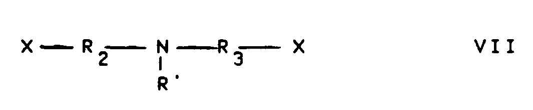 dans laquelle R₂, R₃ et R′ ont la signification donnée précédemment et X représente un groupe labile tel qu'un groupe tosyloxy, mésyloxy ou un atome de chlore, de brome ou d'iode, ou
b) par réaction d'une diamine de formule

   R′ HN―R₁―NH―R′ VIII

   dans laquelle R₁ et R′ ont la signification donnée précédemment,
avec un composé de formule 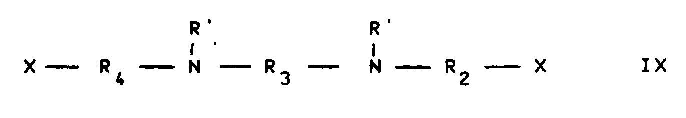
Cette réaction de cyclisation est réalisée avantageusement en présence d'un catalyseur de transfert de phase.

Les polyamines de formule VI peuvent être obtenues à partir de dihydroxylamines selon le schéma suivant : 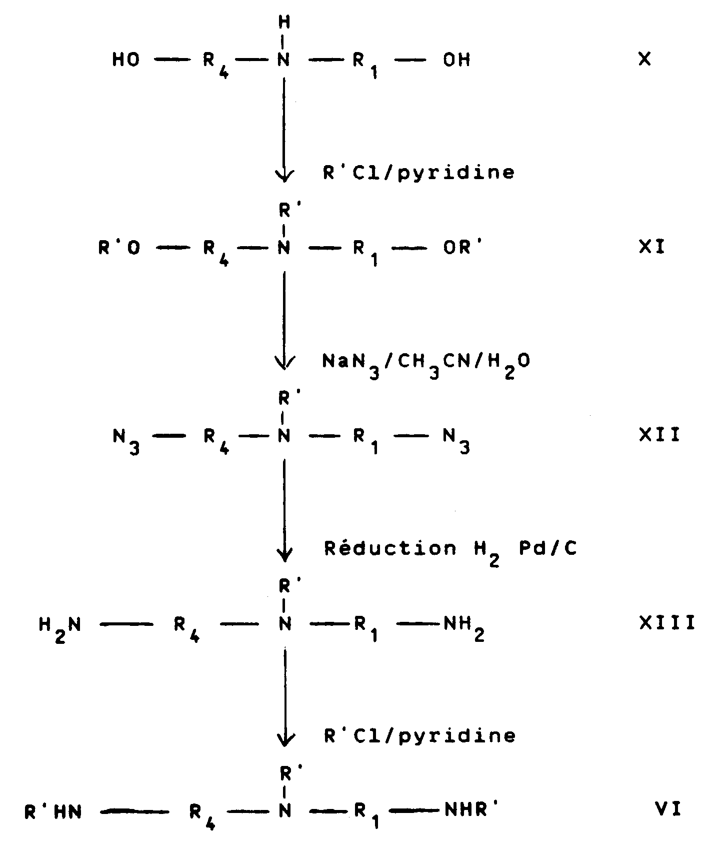
En variante on peut faire réagir sur les composés de formule XI le phtalimide et effectuer une hydrazinolyse pour passer des composés de formule XI aux composés de formule XIII.

La présente invention a en outre pour objet des complexes formés par les ligands de formule I avec des ions métalliques choisis parmi les ions des lanthanides (numéros atomiques 57 à 71), les ions des métaux de transition (numéros atomiques 21 à 29) notamment Mn²⁺, Fe ³⁺ et Cr³⁺, et les ions des métaux de numéros atomiques 55, 56, 82 et 83, ainsi que les sels de ces complexes avec des bases minérales ou organiques pharmaceutiquement acceptables ou des aminoacides basiques.

Dans ces complexes les ions métalliques sont de préférence les ions des lanthanides choisis parmi le gadolinium, l'europium, le dysprosium, et les ions des métaux de transition choisis parmi le fer (Fe³⁺), le manganèse (Mn²⁺) et le chrome.

Comme exemples de sels on peut citer ceux formés avec 1 hydroxyde de sodium, la N-methylglucamine, la diéthanolamine, la lysine et l'arginine.

Les complexes peuvent être obtenus par réaction des ligands avec un sel ou un oxyde des métaux dans un solvant aqueux et éventuellement neutralisation pour former un sel.

Il va de soi que la présente invention englobe non seulement les ligands de formule I et les complexes précédemment définis sous forme de mélange racémique mais également les stéréoisomères de ces ligands et complexes.

Les complexes selon l'invention peuvent en outre être liés à une macromolécule qui est susceptible de se fixer préférentiellement sur un organe. C'est ainsi que les complexes selon l'invention peuvent être liés à des protéines et notamment des anticorps.

Ils peuvent en outre être greffés ou encapsulés notamment dans des liposomes.

Les complexes selon l'invention formés par les ligands de formule I avec des ions paramagnétiques et leurs sels avec des bases pharmaceutiquement acceptables peuvent être utilisés comme agent de contraste en imagerie par résonance magnétique et notamment avec le dysprosium, comme agent de déplacement chimique in vivo.

Les complexes selon l'invention formés par des ligands de formule I avec des ions des lanthanides (numéros atomiques 57 à 71) ou des ions des métaux de numéro atomique 55, 56, 82 et 83 et leurs sels avec des bases pharmaceutiquement acceptables peuvent être utilisés comme produits de contraste en radiographie par rayons X. A cet effet, on préfère en particulier les complexes formés avec les ions des métaux suivants : Gd, Er, Dy, Tb, Ce, La, Ba et Cs.

La présente invention a en conséquence également pour objet une composition de diagnostic administrable à l'homme caractérisé en ce qu'elle comprend au moins un complexe formé par un ligand de formule I avec des ions métalliques choisis parmi les ions des lanthanides, les ions des métaux de transition et les ions des métaux de numéro atomique 55, 56, 82 et 83, ainsi que les sels de ces complexes avec des bases minérales, ou organiques, pharmaceutiquement acceptables ou des aminoacides basiques.

Ces compositions peuvent être constituées notamment par des solutions dans un solvant aqueux physiologiquement acceptable d'un complexe selon l'invention.

Les compositions de diagnostic selon l'invention peuvent être administrées :
- par voie parentérale dont la voie intraveineuse, la voie intra-artérielle, la voie intralymphatique, la voie sous cutanée
- par voie orale
- par voie sous-arachnoïdienne
- par voie intrabronchique sous forme d'aérosol
- par voie intraarticulaire
- localement pour la visualisation des cavités (par exemple utérus).

En imagerie par résonance magnétique, les doses sont très variables selon les voies d'administration.

Pour la voie intraveineuse ou intra-artérielle, la dose est d'environ 0,01 à 2 mM/kg.

Pour la voie orale cette dose peut aller jusqu'à 10 mM/kg.

Pour les autres voies d'administration, les doses utiles sont généralement inférieures à 1 mM/kg et même généralement pour la voie sous-arachnoïdienne inférieure à 0,05 mM/kg.

Dans l'utilisation comme agents de déplacement chimique in vivo et comme agent de contraste en radiologie par rayons X les doses sont les mêmes, sauf par la voie intraveineuse ou intra-artérielle où les doses peuvent être inférieures ou égales à 5 mM/kg.

En outre, les complexes selon l'invention formés par les ligands de formule I avec des ions radioactifs ainsi que leurs sels avec des bases pharmaceutiquement acceptables peuvent être utilisés comme agents de diagnostic ou de thérapie en médecine nucléaire. Des exemples d'ions radioactifs sont des radioisotopes d'éléments tels que le cuivre, le cobalt, le gallium, le germanium, l'indium et surtout le technétium (Tc 99 m).

Les exemples suivants illustrent la préparation des composés selon la présente demande.

Dans ces exemples :
- Les spectres RMM ont été réalisés sur un appareil Varian EM 360 à 60 MHz avec le TMs comme référence interne. Le solvant est CDCl₃ sauf indication contraire.
- Les spectres IR ont été réalisés sur un appareil Perkin-Elmer 1320. Les spectres des solides sont enregistrés sous forme de pastilles de KBr. Dans le cas des liquides (huiles) ils sont enregistrés sans solvant.
- Le terme "tampon" utilisé lors de la chromatographie sur couche mince désigne un mélange NH₄OH 1,5 M et (NH₄)₂CO₃ 1,5 M.
- Les points de fusion ont été enregistrés sur banc Kofler.
- Les termes utilisés lors des dosages en cours de complexation :"absence de Gd³⁺ libre et de ligand libre" s'entendent dans les limites de détection des méthodes utilisées c'est-à-dire <4 ppm et <5 ppm pour Gd³⁺ et ligand respectivement.

### Exemple 1 - Préparation du monopropionamide de l'acide 2-méthyl-1,4,7,10-tétraazacyclododécane-N,N′,N˝,N‴-tétraacétique.

### A - Préparation de l'acide 2-méthyl-1,4,7,10-tétraazacyclododécane-N,N′N˝,N‴-tétraacétique

### a) Préparation du N,N′-ditosyl-1,2-diaminopropane.

Dans un ballon tricol de 1 l équipé d'une agitation magnétique, d'un thermomètre et d'une garde à chlorure, 14,8 g du 1,2-diaminopropane sont solubilisés dans 500 ml de CH₂Cl₂ et 58 cm³ de Et₃N.

En une heure, 80 g de chlorure de tosyle sont introduit par portions. Il est nécessaire de refroidir à l'aide d'un bain de glace pour maintenir une température de 20°C.

Le mélange réactionnel est ensuite agité une nuit à température ambiante.

Le mélange réactionnel est transferré dans une ampoule à décanter de 1 l puis lavé avec 2 x 250 cm³ d eau.

La fraction organique est séchée sur Na₂SO₄, évaporée à sec, puis cristallisée dans l'éther isopropylique.

| | |
|---|---|
| Masse obtenue | = 66 g |
| Rdt | = 86 % |
| Point de fusion | = 98/100°C |

| RMN | |
|---|---|
| 1 ppm doublet (3H) | 3,1 ppm massif (1 H) |
| 2,4 ppm singulet (6H) | 5,5 ppm singulet échangeable (2H) |
| 2,9 ppm doublet (2H) | 7,1 à 7,8 ppm aromatique (8H) |

| CCM | | |
|---|---|---|
| SiO₂ | éluant CH₂Cl₂ 90 | Rf = 0,75 |
| | MeOH 10 | |

### b) Préparation du N,N′-ditosyl-bis (2-tosyloxyethyl) éthylène diamine.

Dans un ballon tricol de 500 cm³ équipé d'un thermomètre, d'une garde à chlorure et d'une agitation magnétique, une solution de 162g de chlorure de tosyle dans 300 ml de pyridine est refroidie à 0°C, à l'aide d'un bain de glace et sel.

A cette température, en 2 h, 29,6g de bis (2-hydroxyéthyl) éthylènediamine sont ajoutés par portions. La température ne doit à aucun moment, excéder 5°C. Le mélange réactionnel est agité 4 h à cette température, laissé 48 h à 6/8°C au réfrigérateur puis 4h à température ambiante.

Le mélange réactionnel est jeté sur 1 l de glace + eau et 300 ml d'HCl concentré. Le produit est extrait avec 500 ml de CH₂Cl₂. Cette phase organique est séchée sur Na₂SO₄ puis évaporée à sec. Le résidu est repris à chaud dans 250 cm³ d'éthanol. Le produit cristallise. Il est essoré sur verre fritté, séché à 60°C 48 h.

| | |
|---|---|
| Masse obtenue | = 107,5 g |
| Rdt | = 70% |
| Point de fusion | = 138/140°C |

RMN
2,4 ppm singulet (12 H)
3,3 ppm singulet + triplet (8 H)
4,2 ppm triplet (4 H)
7,2 à 7,8 ppm massif (16 H)

| CCM | | |
|---|---|---|
| Plaque SiO₂ | éluant toluène 80 | Rf = 0,6 |
| | acétone 20 | |

### c) Préparation du N,N′,N˝,N‴-tétratosyl-2-méthyl-1, 4,7,10-tétraazacyclododécane.

Dans un ballon tricol de 1 l, une solution de 17,5 g de N,N′ ditosyldiamino 1,2-propane dans 500 ml de DMF sec, est agitée 1/4 d'heure à température ambiante. 33 g de Cs₂CO₃ préalablement pulvérisés sont ajoutés en suspension à cette solution. Cette suspension est portée à 55°C à l'aide d'un bain d'huile sous atmosphère inerte.

A cette température, et en 2 h, une solution de 35 g de N,N′-ditosyl-bis (2-tosyloxyethyl) éthylène diamine dans 200 cm³ de DMF est ajoutée goutte à goutte. Après la fin de l'addition, le chauffage est poursuivi pendant 48 h. Le DMF est ensuite éliminé par distillation sous vide. Le résidu est repris dans un mélange eau/CH₂Cl₂.

La phase organique est séchée sur Na₂SO₄. Le solvant est éliminé par distillation à l'évaporateur rotatif.

Le résidu est agité à chaud, dans 200 ml d acétate d'éthyle. Le produit attendu cristallise. Il est essoré, puis séché à 60°C, sous vide 24 h.

| | |
|---|---|
| Masse obtenue | = 22,5 g |
| Rendement | = 61 % |
| Point de fusion | = 274/275°C |

RMN
1 ppm doublet (2 H)
2,2 ppm singulet (12 H)
3 à 3,8 ppm massif (15 H)
7,2 à 7,9 ppm massif (16 H) aromatique

| CCM | | |
|---|---|---|
| SiO₂ | éluant toluène = 80 | Rf = 0,56 |
| | acétone = 20 | |

### d) Préparation du 2-méthyl-1,4,7,10-tetraazacyclododecane.

Dans un tricol de 1 l équipé d'un thermomètre, d'un ballon d'argon et d'une agitation magnétique, une solution de 72,5 g du composé obtenu en c) dans 300 ml d'H₂SO₄ à 98% est chauffée à 100°C pendant 48 h à l'aide d'un bain d'huile sous atmosphère inerte.

Le mélange réactionnel est refroidi à température ambiante et additionné en 1 h à 800 ml d'Et₂O refroidi à 0°C, à l'aide d'un bain d'éthylène glycol et de carboglace.

Le sulfate très hygroscopique précipite. Il est essoré soigneusement sous azote sur un verre fritté, puis solubilisé rapidement dans 200 ml d'eau. Cette solution est alcalinisée (NaOH en pastilles) puis extraite avec 5 x 100 ml de CH₂Cl₂.

Les phases organiques réunies sont séchées sur Na₂SO₄ puis évaporées à sec pour conduire à 15 g de produit brut très visqueux qui cristallise dans le temps.

| | |
|---|---|
| Rendement | = 90% |

| RMN | | |
|---|---|---|
| CDCl₃ | 1,1 ppm | doublet (2 H) |
| Spectre dans + D₂O | 2,7 ppm | 2 singulets en massif (19 H dont 4 échangeables) |

| CCM | | |
|---|---|---|
| Plaque Al₂O₃ | éluant BuOH 50 | Rf = 0,8 |
| | H₂O 25 | |
| | ACOH 11 | |

### e) Préparation du complexe de l'acide 2-méthyl-1,4,7,10-tétraazacyclododécane-N,N′,N˝,N‴-tétraacétique avec 2 KCl.

Dans un ballon tricol de 250 ml, une solution de 34 g d'acide chloroacétique, dans 150 cm³ d'eau, est refroidie à 10°C, dans un bain de glace. A cette température, on ajoute de 20 g de potasse pour salifier l'acide.

Le composé obtenu en d est ensuite solubilisé dans cette solution. Le mélange réactionnel est alors porté à 65°C à l'aide d'un bain d'huile.

A cette température, en 6 h, en maintenant le pH entre 8 et 10, une solution de 20 g de KOH dans 50 cm₃ d'eau est additionnée précautionneusement.

Le chauffage est ensuite poursuivi 72 heures, puis le milieu réactionnel est acidifié à pH = 2,5 à l'aide d'HCl concentré.

Le complexe précipite. Il est essoré sur verre fritté, rincé avec 50 cm₃ d'eau, puis séché à 60°C 18 h à l'étuve.

| | |
|---|---|
| Masse obtenue | = 30 g |
| Rendement | = 66 % |
| Point de fusion | > 300°C |

### f) Purification de l'acide 2-méthyl-1,4,7,10-tétraazacyclododécane-1,4,7,10-tétraacétique.

30 g de complexe obtenu en e sont mis en suspension en présence de 150 cm³ de resine IRA 958 préalablement régénérée.

Après dissolution du complexe, cette suspension est placée en tête d'une colonne contenant 150 cm³ de résine IRA 958.

L'élution est effectuée par une solution d'acide acétique à 5 % dans l'eau.

Les fractions contenant le produit attendu pur sont évaporées à sec afin d'éliminer entièrement l'acide acétique.

| | |
|---|---|
| Masse obtenue | = 18,4 g |
| Rendement | = 89 % |
| Acidité | = 100,3 % (4 équivalents) dosage avec NaOH 0,1 M. |

| CCM | | |
|---|---|---|
| SiO₂ | éluant ACOET 12 | Rf = 0,36 |
| | Isopropanol 35 | |
| | NH₃, H₂O 30 | |

Spectre de masse FAB pic de masse à M + 1 = 419.

### B - Préparation du monopropionamide de l'acide 2-méthyl-1,4,7,10-tétraazacyclododécane-N,N′,N˝,N‴-tétraacétique

L'acide 2-méthyl-1,4,7,10-tétraazacyclododécane-N,N′,N˝,N‴-tétraacétique (5g; 0,0119 mole) est solubilisé dans 50ml d'eau; à cette solution sont ajoutés 1,4g de diméthylaminopyridine (0,0119 mole) et 0,7g de propylamine (0,0118 mole)

Quand le milieu réactionnel est homogène, 3,5g de chlorhydrate de 1-éthyl-3(3-diméthylaminopropyl)carbodiimide ou EDCI (0,0178 mole) sont ajoutés par portions; le pH est maintenu constant entre 4,5 et 5,0 pendant la première heure de réaction.

La solution est laissée sous agitation à température ambiante pendant 24 heures. Puis celle-ci est placée en tête d'une colonne contenant 80 cm³ de résine IRA958 préalablement régénérée.

L'élution est effectuée par une solution d'acide acétique à 5% dans l'eau.

Les fractions contenant le produit attendu et le produit de départ n'ayant pas réagi sont évaporées à sec afin d'éliminer entièrement l'acide acétique.

Après redissolution du mélange dans l'eau, celui-ci est placé en tête d'une colonne contenant 100 cm³ de résine DOWEX 1x8-400 préalablement régénéré. L'élution est effectuée par l'eau.

Les fractions contenant le produit attendu sont évaporées à sec.
Acidité : milieu hydroalcoolique
3 fonctions dosées

| | |
|---|---|
| 1 acidité forte | 97% |
| 2 acidités faibles | 191% |

CCM sur support SiO₂ éluant :

| | |
|---|---|
| Acétate d'éthyle | 12 |
| Isopropanol | 35 |
| NH₄OH | 30 |
| Rf | = 0,5 |

RMM (Brücker AC 200 E); TMS comme référence interne) dans DMSO 0,83 ppm (t) CH₃; 0,96-1,08 ppm (m) 5H CH₂; 1,38-1,48 ppm (m) 2H CH₂; 2 2,5 ppm (s) 2H CH₂; 2,61-3,48 ppm (m) 21H CH₂.

### Exemple 2 : Préparation d'une solution du complexe de gadolinium du monopropionamide de l'acide 2-méthyl-1,4,7,10-tétraazacyclododécane - N,N′,N˝,N‴-tétraacétique

On dissout 50 mmoles du composé obtenu à l'exemple 1 et 25 mmoles d'oxyde de gadolinium dans 250 ml d'eau bidistillée et dégazée à 70°C. Au bout d'une journée, la complexation est terminée. On filtre sur une membrane millipore 0,22 »m et on évapore jusqu'à la concentration maximale.

## Revendications (Revendications pour l'(les) Etat(s) contractant(s) suivant(s): AT, BE, CH, DE, FR, GB, GR, IT, LI, LU, NL, SE)

1. Ligands de formule : 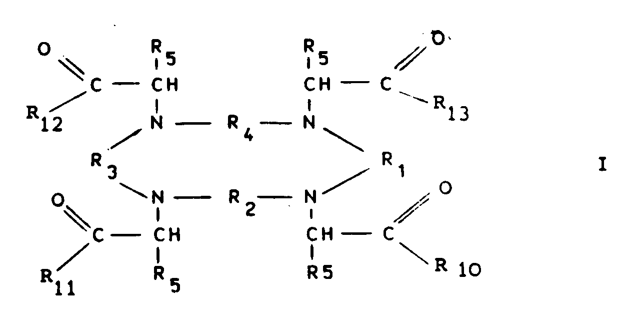 dans laquelle
R₁ représente un radical de formule 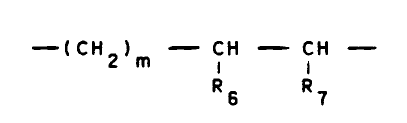 R₆ étant choisi parmi un groupe alkyle en C₁-C₁₈, un groupe hydroxyalkyle en C₁-C₆ et un groupe polyhydroxyalkyle en C₁-C₆
m = 0 ou 1,
R₇ étant choisi parmi un atome d'hydrogène, un groupe alkyle en C₁-C₁₄, un groupe hydroxyalkyle en C₁-C₆ et un groupe polyhydroxyalkyle en C₁-C₆,
R₂, R₃, R₄ identiques ou différents représentent un radical de formule 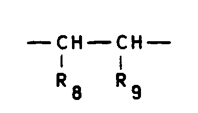 R₈, R₉ identiques ou différents étant choisis parmi un atome d'hydrogène, un groupe alkyle en C₁-C₁₄, une groupe hydroxalkyle en C₁-C₆ et un groupe polyhydroxyalkyle en C₁-C₆,
R₅ est choisi parmi un atome d'hydrogène, un groupe alkyle en C₁-C₁₄, un groupe hydroxyalkyle en C₁-C₆ et un groupe polyhydroxyalkyle en C₁-C₆,
R₁₀, R₁₁, R₁₂, R₁₃ sont choisis indépendamment l'un de l'autre parmi les groupes hydroxy et les groupes de formule 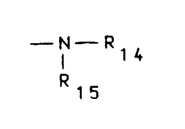 R₁₄ et R₁₅ identiques ou différents étant choisis parmi un atome d'hydrogène, un groupe alkyle en C₁-C₁₄, une groupe hydroxyalkyle en C₁-C₆ et un groupe polyhydroxyalkyle en C₁-C₆,
au moins l'un des groupes R₁₀, R₁₁, R₁₂ et R₁₃ étant un groupe de formule 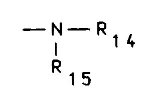 ainsi que leurs sels.

2. Composé selon la revendication 1 qui est le monopropylamide de l'acide 2-méthyl-1,4,7,10-tétraazacyclododécane-N,N',N'',N'''-tétraacétique.

3. Procédé de préparation d'un composé selon la revendication 1, caractérisé en ce que l'on fait réagir un composé de formule 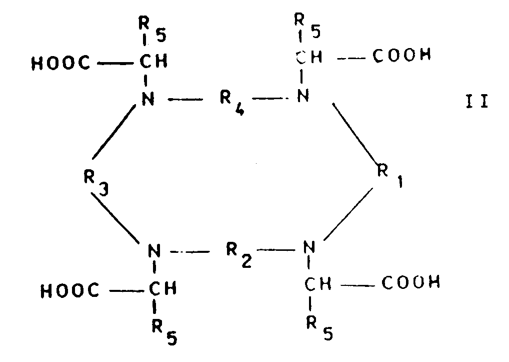 dans laquelle R₁, R₂, R₃, R₄, R₅ ont la signification donnée précédemment, avec une amine de formule 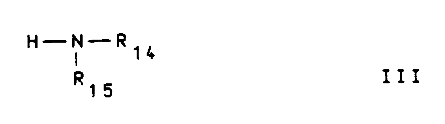 R₁₄ et R₁₅ ayant la signification donnée précédemment.

4. Complexes formés par des ligands de formule I selon la revendication 1, avec des ions métalliques choisis parmi les ions des lanthanides (numéros atomiques 57 à 71), les ions des métaux de transition (numéros atomiques 21 à 29) et les ions des métaux de numéro atomique 55, 56, 82 et 83, ainsi que les sels de ces complexes avec des bases minérales ou organiques pharmaceutiquement acceptables ou des aminoacides basiques.

5. Complexes selon la revendication 4, dans lesquels l'ion métallique est choisi parmi le gadolinium, l'europium, le dysprosium, le fer (Fe³⁺) et le manganèse (Mn²⁺) et le chrome.

6. Composition de diagnostic administrable à l'homme, caractérisée en ce qu'elle comprend au moins un complexe selon l'une quelconque des revendications 4 et 5.

7. Composition selon la revendication 6, constituée par une solution dans un solvant aqueux du complexe.

## Revendications (Revendications pour l'(les) Etat(s) contractant(s) suivant(s): ES)

1. Procédé de préparation de ligands de formule : 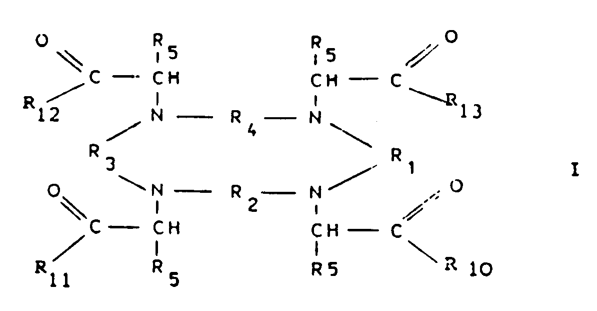 dans laquelle
R₁ représente un radical de formule 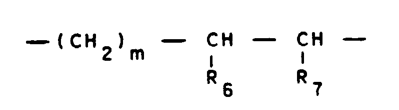 R₆ étant choisi parmi un groupe alkyle en C₁-C₁₈, un groupe hydroxyalkyle en C₁-C₆ et un groupe polyhydroxyalkyle en C₁-C₆
m = 0 ou 1,
R₇ étant choisi parmi un atome d'hydrogène, un groupe alkyle en C₁-C₁₄, un groupe hydroxyalkyle en C₁-C₆ et un groupe polyhydroxyalkyle en C₁-C₆,
R₂, R₃, R₄ identiques ou différents représentent un radical de formule 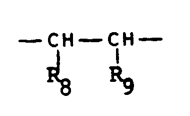 R₈, R₉ identiques ou différents étant choisis parmi un atome d'hydrogène, un groupe alkyle en C₁-C₁₄, une groupe hydroxyalkyle en C₁-C₆ et un groupe polyhydroxyalkyle en C₁-C₆,
R₅ est choisi parmi un atome d'hydrogène, un groupe alkyle en C₁-C₁₄, un groupe hydroxyalkyle en C₁-C₆ et un groupe polyhydroxyalkyle en C₁-C₆,
R₁₀, R₁₁, R₁₂, R₁₃ sont choisis indépendamment l'un de l'autre parmi les groupes hydroxy et les groupes de formule 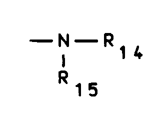 R₁₄ et R₁₅ identiques ou différents étant choisis parmi un atome d'hydrogène, un groupe alkyle en C₁-C₁₄, une groupe hydroxyalkyle en C₁-C₆ et un groupe polyhydroxyalkyle en C₁-C₆,
au moins l'un des groupes R₁₀, R₁₁, R₁₂, et R₁₃ étant un groupe de formule 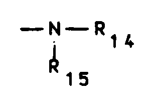 ainsi que leurs sels, caractérisé en ce que l'on fait réagir un composé de formule 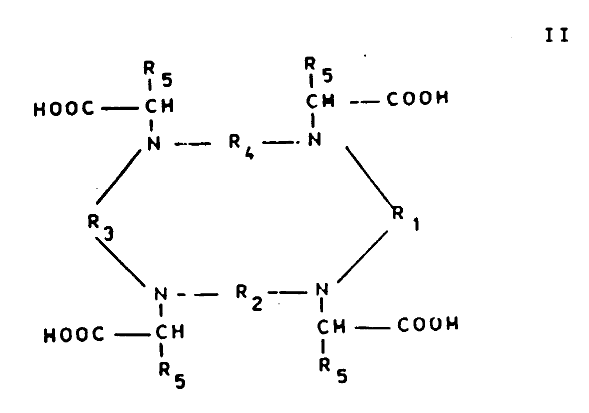 dans laquelle R₁, R₂, R₃, R₄, R₅ ont la signification donnée précédemment, avec une amine de formule 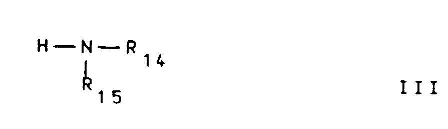 R₁₄ et R₁₅ ayant la signification donnée précédemment.

2. Procédé selon la revendication 1 caractérisé en ce que l'on prépare le monopropylamide de l'acide 2-méthyl- 1,4,7,10-tétraazacyclododécane-N,N',-N'',N'''- tétraacétique.

3. Procédé de préparation de complexes formés par des ligands de formule I tels que définis à la revendication 1, avec des ions métalliques choisis parmi les ions des lanthanides (numéros atomiques 57 à 71), les ions des métaux de transition (numéros atomiques 21 à 29) et les ions des métaux de numéro atomique 55, 56, 82 et 83, ainsi que les sels de ces complexes avec des bases minérales ou organiques pharmaceutiquement acceptables ou des aminoacides basiques caractérisé en ce que l'on fait réagir le ligand avec un sel ou un oxyde des métaux dans un solvant aqueux et éventuellement on neutralise pour former un sel.

4. Procédé de préparation de complexes selon la revendication 3, dans lequel l'ion métallique est choisi parmi le gadolinium, l'europium, le dysprosium, le fer (Fe³⁺) le manganèse (Mn²⁺) et le chrome.

5. Procédé de préparation d'une composition de diagnostic administrable à l'homme, caractérisée en ce que l'on met au moins un complexe tel que défini à la revendication 3 sous une forme pharmaceutiquement acceptable.

## Claims (Claims for the following Contracting State(s): AT, BE, CH, DE, FR, GB, GR, IT, LI, LU, NL, SE)

1. Ligands of the formula: 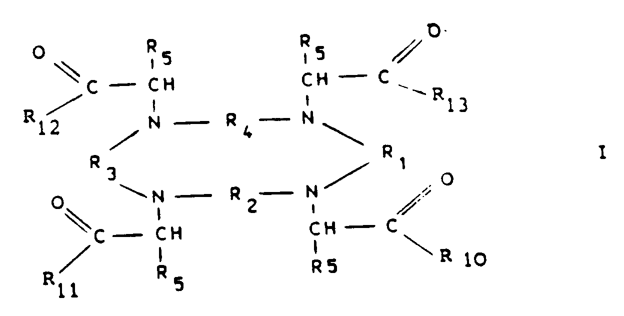 in which
R₁ represents a radical of the formula: 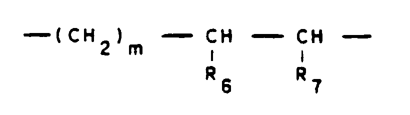
- R₆ being selected from amongst a C₁ - C₁₈ alkyl group, a C₁ - C₆ hydroxyalkyl group and a C₁ - C₆ polyhydroxyalkyl group,
- m = 0 or 1,
- R₇ being selected from amongst a hydrogen atom, a C₁ - C₁₄ alkyl group, a C₁ - C₆ hydroxyalkyl group and a C₁ - C₆ polyhydroxyalkyl group,
R₂, R₃ and R₄ are identical or different and represent a radical of the formula: 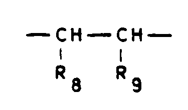
- R₈ and R₉, which are identical or different, being selected from amongst a hydrogen atom, a C₁ - C₁₄ alkyl group, a C₁ - C₆ hydroxyalkyl group and a C₁ - C₆ polyhydroxyalkyl group,
R₅ is selected from amongst a hydrogen atom, a C₁ - C₁₄ alkyl group, a C₁ - C₆ hydroxyalkyl group and a C₁ - C₆ polyhydroxyalkyl group, and R₁₀, R₁₁, R₁₂ and R₁₃ are selected independently from one another, from amongst hydroxy groups and groups of the formula 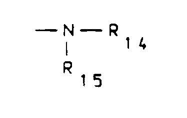
- R₁₄ and R₁₅, which are identical or different, being selected from amongst a hydrogen atom, a C₁ - C₁₄ alkyl group, a C₁ - C₆ hydroxyalkyl group and a C₁ - C₆ polyhydroxyalkyl group,
at least one of the groups R₁₀, R₁₁, R₁₂ and R₁₃ being a group of the formula: 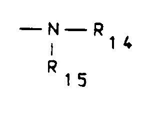 and also salts thereof.

2. A compound in accordance with claim 1, which is the monopropylamide of 2-methyl-1,4,7,10-tetraazacyclododecane-N,N',N'',N'''-tetraacetic acid.

3. A process for preparing a compound in accordance with claim 1, **characterised in that** a compound of the formula: 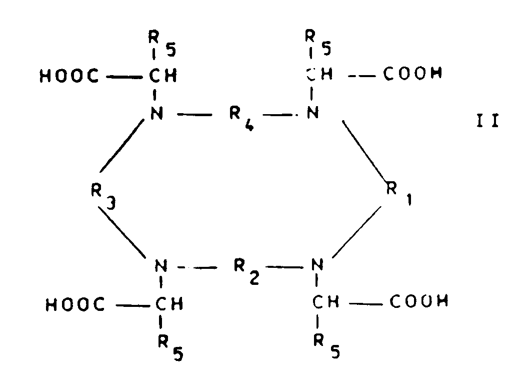 wherein R₁, R₂, R₃, R₄ and R₅ have the meaning previously stated, is made to react with an amine of the formula 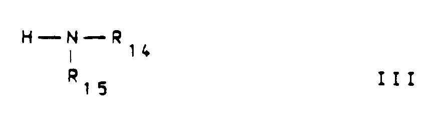 R₁₄ and R₁₅ having the meaning previously stated.

4. Complexes formed by the ligands of formula I in accordance with claim 1, with metal ions selected from amongst lanthanide ions (atomic numbers 57 to 71), transition metal ions (atomic numbers 21 to 29) and ions of metals with the atomic numbers 55, 56, 82 and 83, and also salts of these complexes with pharmaceutically acceptable mineral or organic bases, or basic aminoacids.

5. Complexes in accordance with claim 4, wherein the metal ion is selected from amongst gadolinium, europium, dysprosium, iron (Fe³⁺), manganese (Mn²⁺) and chromium.

6. A diagnostic composition which can be administered to man, **characterised in that** it comprises at least one complex in accordance with either one of claims 4 and 5.

7. A composition in accordance with claim 6, formed by a solution of the complex in an aqueous solvent.

## Claims (Claims for the following Contracting State(s): ES)

1. A process for preparation of ligands of the formula: 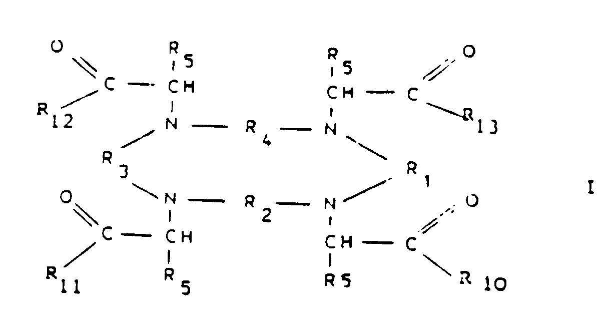 in which
R₁ represents a radical of the formula: 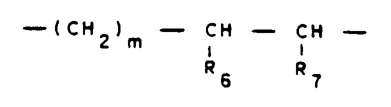
- R₆ being selected from amongst a C₁ - C₁₈ alkyl group, a C₁ - C₆ hydroxyalkyl group and a C₁ - C₆ polyhydroxyalkyl group,
- m = 0 or 1,
- R₇ being selected from amongst a hydrogen atom, a C₁ - C₁₄ alkyl group, a C₁ - C₆ hydroxyalkyl group and a C₁ - C₆ polyhydroxyalkyl group,
R₂, R₃ and R₄ are identical or different and represent a radical of the formula: 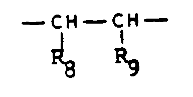
- R₈ and R₉, which are identical or different, being selected from amongst a hydrogen atom, a C₁ - C₁₄ alkyl group, a C₁ - C₆ hydroxyalkyl group and a C₁ - C₆ polyhydroxyalkyl group,
R₅ is selected from amongst a hydrogen atom, a C₁ - C₁₄ alkyl group, a C₁ - C₆ hydroxyalkyl group and a C₁ - C₆ polyhydroxyalkyl group, and R₁₀, R₁₁, R₁₂ and R₁₃ are selected independently from one another, from amongst hydroxy groups and groups of the formula 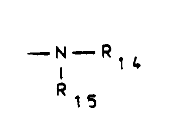
- R₁₄ and R₁₅, which are identical or different, being selected from amongst a hydrogen atom, a C₁ - C₁₄ alkyl group, a C₁ - C₆ hydroxyalkyl group and a C₁ - C₆ polyhydroxyalkyl group,
at least one of the groups R₁₀, R₁₁, R₁₂ and R₁₃ being a group of the formula: 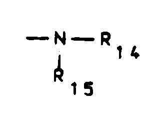 and also salts thereof, **characterised in that** a compound of the formula: 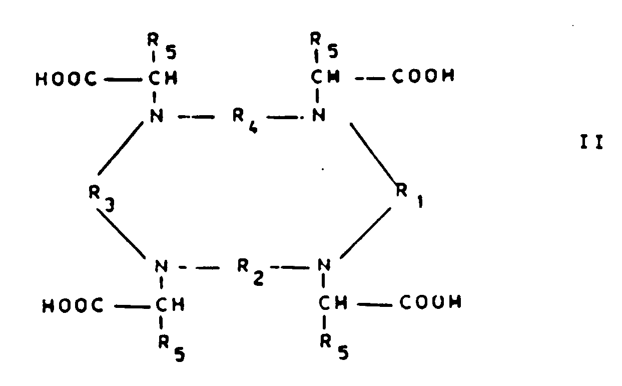 wherein R₁, R₂, R₃, R₄ and R₅ have the meaning previously stated, is made to react with an amine of the formula 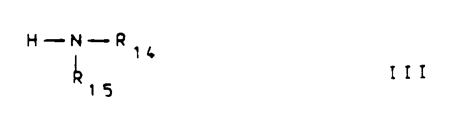 R₁₄ and R₁₅ having the meaning previously stated.

2. A process in accordance with claim 1, **characterised in that** the monopropylamide of 2-methyl-1,4,7,10-tetraazacyclododecane-N,N',N'',N'''-tetraacetic acid is prepared.

3. A process for preparing complexes formed by the ligands of formula I as defined in claim 1, with metal ions selected from amongst lanthanide ions (atomic numbers 57 to 71), transition metal ions (atomic numbers 21 to 29) and ions of metals with the atomic numbers 55, 56, 82 and 83, and also salts of these complexes with pharmaceutically acceptable mineral or organic bases, or basic aminoacids, **characterised in that** the ligand is made to react with a salt or an oxide of the metals in an aqueous solvent, and neutralising is optionally carried out to form a salt.

4. A process for preparing complexes in accordance with claim 3, in which the metal ion is selected from amongst gadolinium, europium, dysprosium, iron (Fe³⁺), manganese (Mn²⁺) and chromium.

5. A process for preparing a diagnostic composition which can be administered to man, **characterised in that** at least one complex as defined in claim 3 is put into a pharmaceutically acceptable form.

## Patentansprüche (Patentansprüche für folgende(n) Vertragsstaat(en): AT, BE, CH, DE, FR, GB, GR, IT, LI, LU, NL, SE)

1. Liganden der Formel 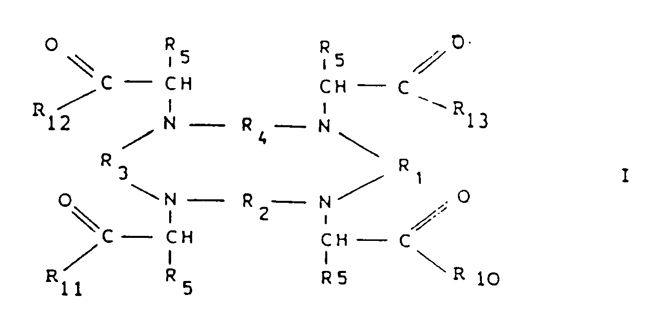 in der bedeuten:
R₁ einen Rest der Formel 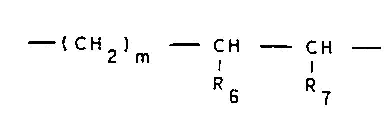
R₆ einen Rest, ausgewählt aus einer C₁-C₁₈-Alkylgruppe, einer C₁-C₆-Hydroxyalkylgruppe und einer C₁-C₆-Polyhydroxyalkylgruppe,
m die Zahl 0 oder 1,
R₇ einen Rest, ausgewählt aus einem Wasserstoffatom, einer C₁-C₁₄-Alkylgruppe, einer C₁-C₆-Hydroxyalkylgruppe und einer C₁-C₆-Polyhydroxyalkylgruppe,
R₂, R₃ und R₄, die identisch oder verschieden sind, einen Rest der Formel 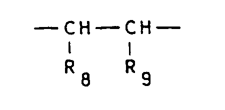
R₈, R₉, die identisch oder verschieden sind, einen Rest, ausgewählt aus einem Wasserstoffatom, einer C₁-C₁₄-Alkylgruppe, einer C₁-C₆-Hydroxyalkylgruppe und einer C₁-C₆-Polyhydroxyalkylgruppe,
R₅ einen Rest, ausgewählt aus einem Wasserstoffatom, einer C₁-C₁₄-Alkylgruppe, einer C₁-C₆-Hydroxyalkylgruppe und einer C₁-C₆-Polyhydroxyalkylgruppe,
R₁₀, R₁₁, R₁₂, R₁₃ unabhängig voneinander ausgewählt werden aus Hydroxygruppen und Gruppen der Formel 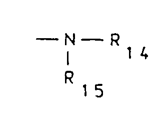
R₁₄ und R₁₅, die identisch oder verschieden sind, Reste, die ausgewählt werden aus einem Wasserstoffatom, einer C₁-C₁₄-Alkylgruppe, einer C₁-C₆-Hydroxyalkylgruppe und einer C₁-C₆-Polyhydroxyalkylgruppe,
wobei mindestens eine der Gruppen R₁₀, R₁₁, R₁₂ und R₁₃ eine Gruppe der Formel darstellt 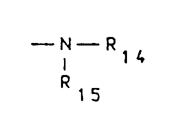 sowie ihre Salze.

2. Verbindung nach Anspruch 1, bei der es sich handelt um das Monopropylamid der 2-Methyl-1,4,7,10-tetraazacyclododecan-N,N',N'',N'''-tetraessigsäure.

3. Verfahren zur Herstellung einer Verbindung nach Anspruch 1, dadurch gekennzeichnet, daß man eine Verbindung der Formel 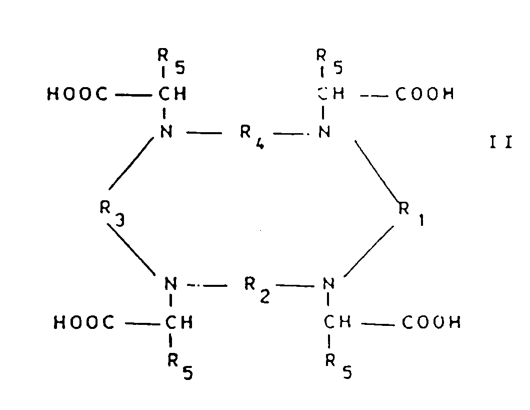 in der R₁, R₂, R₃, R₄ und R₅ die oben angegebenen Bedeutungen haben,
mit einem Amin der Formel (III) reagieren läßt 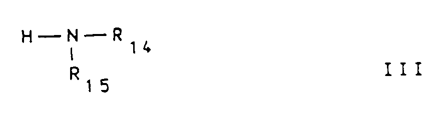 worin R₁₄ und R₁₅ die oben angegebene Bedeutung haben.

4. Komplexe, die gebildet sind aus Liganden der Formel (I) nach Anspruch 1 und Metallionen, ausgewählt aus den Ionen der Lanthanide (Atomzahlen Nr. 57 bis 71), den Ionen der Übergangsmetalle (Atomzahlen Nr. 21 bis 29) und den Ionen von Metallen mit der Atomzahl 55, 56, 82 und 83, sowie die Salze dieser Komplexe mit pharmazeutisch akzeptablen Mineralbasen oder organischen Basen oder mit basichen Aminosäuren.

5. Komplexe nach Anspruch 4, in denen das Metallion ausgewählt wird aus der Gruppe Gadolinium, Europium, Dysprosium, Eisen (Fe³⁺), Mangan (Mn²⁺) und Chrom.

6. An Menschen verabreichbare Diagnosezusammensetzung, dadurch gekennzeichnet, daß sie mindestens einen Komplex nach einem der Ansprüche 4 und 5 enthält.

7. Zusammensetzung nach Anspruch 6, die besteht aus einer Lösung des Komplexes in einem wäßrigen Lösungsmittel.

## Patentansprüche (Patentansprüche für folgende(n) Vertragsstaat(en): ES)

1. Verfahren zur Herstellung von Liganden der Formel 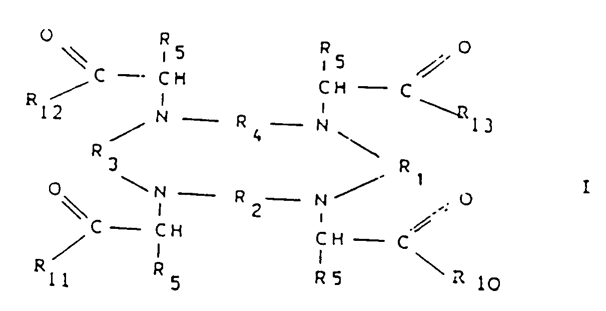 in der bedeuten:
R₁ einen Rest der Formel 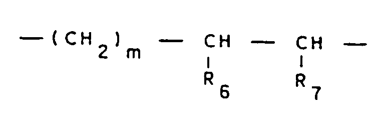
R₆ einen Rest, ausgewählt aus einer C₁-C₁₈-Alkylgruppe, einer C₁-C₆-Hydroxyalkylgruppe und einer C₁-C₆-Polyhydroxyalkylgruppe,
m die Zahl 0 oder 1,
R₇ einen Rest, ausgewählt aus einem Wasserstoffatom, einer C₁-C₁₄-Alkylgruppe, einer C₁-C₆-Hydroxyalkylgruppe und einer C₁-C₆-Polyhydroxyalkylgruppe,
R₂, R₃ und R₄, die identisch oder verschieden sind, einen Rest der Formel 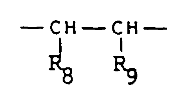
R₈, R₉, die identisch oder verschieden sind, einen Rest, ausgewählt aus einem Wasserstoffatom, einer C₁-C₁₄-Alkylgruppe, einer C₁-C₆-Hydroxyalkylgruppe und einer C₁-C₆-Polyhydroxyalkylgruppe,
R₅ einen Rest, ausgewählt aus einem Wasserstoffatom, einer C₁-C₁₄-Alkylgruppe, einer C₁-C₆-Hydroxyalkylgruppe und einer C₁-C₆-Polyhydroxyalkylgruppe,
R₁₀, R₁₁, R₁₂, R₁₃ unabhängig voneinander ausgewählt werden aus Hydroxygruppen und Gruppen der Formel 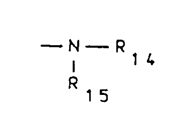
R₁₄ und R₁₅, die identisch oder verschieden sind, Reste, die ausgewählt werden aus einem Wasserstoffatom, einer C₁-C₁₄-Alkylgruppe, einer C₁-C₆-Hydroxyalkylgruppe und einer C₁-C₆-Polyhydroxyalkylgruppe,
wobei mindestens eine der Gruppen R₁₀, R₁₁, R₁₂ und R₁₃ eine Gruppe der Formel darstellt 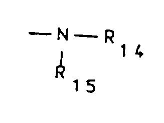 sowie ihrer Salze, dadurch gekennzeichnet, daß man eine Verbindung der Formel 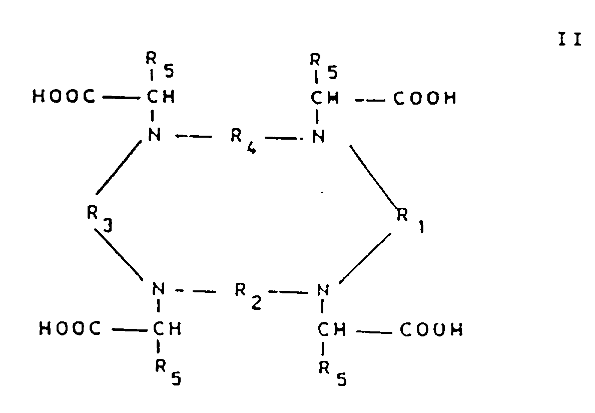 in der R₁, R₂, R₃, R₄ und R₅ die oben angegebenen Bedeutungen haben,
mit einem Amin der Formel (III) reagieren läßt 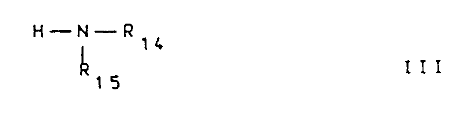 worin R₁₄ und R₁₅ die oben angegebene Bedeutung haben.

2. Verfahren nach Anspruch 1, dadurch gekennzeichnet, daß man das Monopropylamid der 2-Methyl-1,4,7,10-tetraazacyclododecan-N,N',N'',N'''-tetraessigsäure herstellt.

3. Verfahren zur Herstellung von Komplexen, die gebildet sind aus Liganden der Formel (I), wie sie im Anspruch 1 definiert sind, und Ionen von Metallen, ausgewählt aus den Ionen der Lanthanide (Atomzahlen Nr. 57 bis 71), den Ionen der Übergangsmetalle (Atomzahlen Nr. 21 bis 29) und den Ionen von Metallen mit der Atomzahl 55, 56, 82 und 83, sowie der Salze dieser Komplexe mit pharmazeutisch akzeptablen Mineralbasen oder organischen Basen oder mit basichen Aminosäuren, dadurch gekennzeichnet, daß man den Liganden mit einem Salz oder einem Oxid der Metalle in einem wäßrigen Lösungsmittel reagieren läßt und gegebenenfalls neutralisiert zur Bildung eines Salzes.

4. Verfahren zur Herstellung von Komplexen nach Anspruch 3, in denen das Metallion ausgewählt wird aus der Gruppe Gadolinium, Europium, Dysprosium, Eisen (Fe³⁺), Mangan (Mn²⁺) und Chrom.

5. Verfahren zur Herstellung einer an Menschen verabreichbaren Diagnosezusammensetzung, dadurch gekennzeichnet, daß man mindestens einen Komplex, wie er in Anspruch 3 definiert ist, in eine pharmazeutisch akzeptable Form überführt.
